# EUROPÄISCHE PATENTSCHRIFT

(11) **EP 3 536 226 B1**
(45) Veröffentlichungstag und Bekanntmachung des Hinweises auf die Patenterteilung: **17.02.2021**
(21) Anmeldenummer: 19160789.4
(22) Anmeldetag: 05.03.2019
(51) Int. Cl.: A61B 5/00, A61B 5/11, A61B 1/00

(54) **MEDIZINISCHE VORRICHTUNG, VERFAHREN ZUR PRÄDIKTION EINES AUFTREFFORTS UND COMPUTERPROGRAMM**
MEDICAL DEVICE, METHOD OF PREDICTING AN IMPACT POINT AND COMPUTER PROGRAM
DISPOSITIF MÉDICAL, PROCÉDÉ DE PRÉDICTION D'UN POINT DE CONTACT ET PROGRAMME INFORMATIQUE

(30) Priorität: 09.03.2018 DE 102018105454
(43) Veröffentlichungstag der Anmeldung: 11.09.2019
(73) Patentinhaber: Medizinische Hochschule Hannover, 30625 Hannover (DE); Gottfried Wilhelm Leibniz Universität Hannover, 30167 Hannover (DE)
(72) Erfinder: KAHRS, Lüder Alexander, Etobicoke, ON M8V0C8 (CA); KUNDRAT, Dennis, 31832 Springe (DE); PTOK, Martin, 30938 Burgwedel-Engensen (DE); FAST, Jacob Friedemann, 30171 Hannover (DE)
(74) Vertreter: Gramm, Lins & Partner Patent- und Rechtsanwälte PartGmbB

(56) Entgegenhaltungen:
- US-A1- 2015 305 610
- US-A1- 2016 081 591
- PTOK M ET AL: "Deliberate release of the laryngeal adductor reflex via microdroplet impulses", HNO. HALS-, NASEN-, OHRENAERZTE, SPRINGER VERLAG, BERLIN, DE, Bd. 64, Nr. 3, 17. Februar 2016 (2016-02-17), Seiten 149-155, XP035801130, ISSN: 0017-6192, DOI: 10.1007/S00106-016-0130-1 [gefunden am 2016-02-17]
- JACOB FRIEDEMANN FAST ET AL: "Towards microprocessor-based control of droplet parameters for endoscopic laryngeal adductor reflex triggering", CURRENT DIRECTIONS IN BIOMEDICAL ENGINEERING, Bd. 3, Nr. 2, 7. September 2017 (2017-09-07), Seiten 239-243, XP55606149, DOI: 10.1515/cdbme-2017-0050
- SEN A K ET AL: "Aerosol Formation in Electrospray Ionization Using a Microfluidic Emitter", IEEE SENSORS JOURNAL, IEEE SERVICE CENTER, NEW YORK, NY, US, Bd. 11, Nr. 10, 1. Oktober 2011 (2011-10-01), Seiten 2335-2341, XP011480170, ISSN: 1530-437X, DOI: 10.1109/JSEN.2011.2135849
- PARIENTA D ET AL: "Theoretical analysis of the motion and evaporation of exhaled respiratory droplets of mixed composition", JOURNAL OF AEROSOL SCIENCE, ELSEVIER, AMSTERDAM, NL, Bd. 42, Nr. 1, 1. Januar 2011 (2011-01-01) , Seiten 1-10, XP027579191, ISSN: 0021-8502 [gefunden am 2010-12-29]

## Beschreibung

Die Erfindung betrifft eine medizinische Vorrichtung zum gezielten Abschuss eines Fluidtropfens in ein Zielgebiet eines Lebewesens. Die Erfindung betrifft außerdem ein Verfahren zur Prädiktion eines Auftrefforts eines mit einer solchen Einrichtung abgeschossenen Fluidtropfens sowie ein Computerprogramm zur Durchführung eines solchen Verfahrens.

In verschiedenen Bereichen der Medizin/Tiermedizin besteht ein Bedarf, Fluidtropfen mittels einer Abschuss-Vorrichtung in ein Zielgebiet eines Lebewesens, z.B. eines Menschen oder eines Tiers, abzuschießen. Mit einem solchen abgeschossenen Fluidtropfen kann beispielsweise ein gezielter örtlicher Reiz im Zielgebiet erzeugt werden, z. B. im Bereich der Rachenschleimhaut, um Reflexe im Kehlkopfbereich zu evaluieren. Weitere Anwendungsgebiete einer solchen medizinischen Vorrichtung liegen beispielsweise im Bereich Drug Delivery, zur chemischen Kauterisation oder zur intrauterinen Insemination.

Im Bereich der Atemwege kann eine solche medizinische Vorrichtung beispielsweise zur Vorbeugung von Atemwegserkrankungen genutzt werden. Die tieferen Atemwege werden beim gesunden Lebewesen durch reflexhafte Vorgänge vor Fremdstoffen geschützt. Es wird vermutet, dass ein gestörter Ablauf dieser reflexhaften Vorgänge ein erhöhtes Risiko für die Aspiration von Fremdkörpern zur Folge hat und dies zur Auslösung einer Lungenentzündung (Aspirationspneumonie) führen kann. Aus der US 2016/0081591 A1 sind ein System und ein Verfahren zur Untersuchung des laryngealen Schutzreflexes bekannt.

Aus der US 2015/0305610 A1 sind ein System und ein Verfahren zur Messung von Reflexantworten von Gewebe bekannt. Aus der Veröffentlichung Deliberate release of the laryngeal adductor reflex via microdroplet impulse (Ptok M. et al., HNO. Hals-, Nasen-, Ohrenaerzte, Springer Verlag Berlin, DE, Bd. 64, Nr. 3, 17. Februar 2016, Seiten 149-155) ist eine gezielte Auslösung des laryngealen Adduktionsreflexes per Mikrotröpfchenabgabe bekannt.

Der Erfindung liegt die Aufgabe zugrunde, eine solche medizinische Vorrichtung praxistauglicher und anwendungsfreundlicher zu gestalten. Ferner soll ein entsprechendes Verfahren zum Betrieb einer solchen medizinischen Vorrichtung sowie ein entsprechendes Computerprogramm angegeben werden.

Diese Aufgabe wird gelöst durch eine medizinische Vorrichtung zum gezielten Abschuss eines Fluidtropfens in ein Zielgebiet eines Lebewesens, wobei die medizinische Vorrichtung aufweist:
a) ein optisches Aufnahmesystem, wie z.B. eine endoskopische Stereooptik, zur automatisierten optischen Erfassung des Zielgebiets,
b) eine mechatronisch regelbare oder geregelte Tropfenabschuss-Vorrichtung, durch die der Fluidtropfen abschießbar ist,
c) eine Flugbahn-Prädiktionseinrichtung, die zur Prädiktion der Flugbahn eines mittels der Tropfenabschuss-Vorrichtung abzuschießenden Fluidtropfens sowie zur Anzeige des voraussichtlichen Auftreffortes des Fluidtropfens im Zielgebiet eingerichtet ist,
dadurch gekennzeichnet, dass die Flugbahn-Prädiktionseinrichtung einen Rechner aufweist und dazu eingerichtet ist, rechnergesteuert anhand gespeicherter Flugbahn-Charakterisierungsdaten eine Flugbahn-Trajektorie des abzuschießenden Fluidtropfens zu bestimmen und unter Nutzung von Bildinformationen des optischen Aufnahmesystems einen Auftreffbereich des Fluidtropfens im Zielgebiet abzuschätzen und überlagert mit einer Darstellung des Zielgebiets auf einem Anzeigegerät anzuzeigen.

Die erfindungsgemäße Vorrichtung weist eine Reihe von Vorteilen auf. So ist durch die endoskopische Stereooptik die Möglichkeit der automatisierten optischen Erfassung des Zielgebiets gegeben, wobei auch Tiefeninformationen (dreidimensionale Bildinformationen) bereitgestellt werden können und für die weitere Verarbeitung genutzt werden können. Dies ermöglicht die Zielpunktprädiktion und damit eine vollständig automatische Durchführung beispielsweise der Bestimmung der Latenzzeit zwischen der Stimulation eines definierten Zielareals im Kehlkopf und der eintretenden Adduktionsbewegung der Stimmlippen. Bisher konnten solche präzisen Untersuchungen mit quantitativen Ergebnissen nicht durchgeführt werden.

Das optische Aufnahmesystem kann auch andere optische Erfassungsmittel, alternativ oder zusätzlich zur endoskopischen Stereooptik, aufweisen, wie z.B. optische Kohärenztomographie oder ein nach dem Time-of-Flight-Verfahren arbeitendes System. Je nach verwendeter Anzeigeeinrichtung zur Anzeige des voraussichtlichen Auftrefforts des Fluidtropfens kann das optische Aufnahmesystem zur Bereitstellung dreidimensionaler Bildinformationen, aus denen die Tiefeninformationen gewonnen werden können, geeignet sein, oder solche Daten nicht bereitstellen. Im letztgenannten Fall kann die geeignete dreidimensionale Visualisierung durch ein zur dreidimensionalen Darstellung geeignetes Anzeigegerät erfolgen, z.B. durch eine 3D-Brille oder einen 3D-Monitor.

Das optische Aufnahmesystem, insbesondere die endoskopische Stereooptik, kann als flexible oder als starre Optik ausgebildet sein, oder als Kombination daraus. Beispielsweise kann das optische Aufnahmesystem einen Bildleiter oder einen Chip-onthe-Tip aufweisen.

Es ist hierbei vorteilhaft, wenn das optische Aufnahmesystem für eine Aufzeichnung von Bildsequenzen mit hoher Aufnahmerate eingerichtet ist, bspw. wenigstens 60 Frames pro Sekunde oder wenigstens 100 Frames pro Sekunde. Hierdurch ist eine besonders präzise Bestimmung der Latenzzeit einer Stimulationsantwort möglich. Die räumliche Darstellung kann bspw. über eine Stereodatenbrille erfolgen.

Die Erfindung ermöglicht es somit, dass ein definiertes Zielareal exakt stimuliert wird. Hierdurch wird es erst möglich, dass die Latenz zwischen Stimulation und Stimulationsantwort mit hoher Güte quantitativ bestimmt werden kann.

Durch die mechatronisch regelbare oder geregelte Tropfenabschuss-Vorrichtung kann ein gleichbleibender Tropfenimpuls und somit eine gleichbleibende Stimulationsstärke durch den abgeschossenen Fluidtropfen gewährleistet werden. Eine manuelle Einstellung oder Regelung durch den Anwender kann entfallen, so dass auch in dieser Hinsicht das System wesentlich anwenderfreundlicher und einfacher zu nutzen wird.

Durch die automatische Prädiktion der Flugbahn mittels der Flugbahn-Prädiktionseinrichtung ist ein zuverlässiges Applizieren des Fluidtropfens an der gewünschten Stelle im Zielgebiet möglich. Bisher erforderliche, manuelle Schritte können hiermit eingespart werden. Durch die automatische Prädiktion der Flugbahn ist eine sichere Einbringung des Fluidtropfens im gewünschten Zielgebiet möglich. Da die Tropfenabschuss-Vorrichtung mechatronisch regelbar oder geregelt ist, kann ein variierender Einfluss der Abschussparameter des Fluidtropfens vermieden werden, so dass auch in dieser Hinsicht die Genauigkeit der Prädiktion der Flugbahn verbessert werden kann.

Die endoskopische Stereooptik kann beispielsweise Lichtleitkörper oder Lichtleitkabel aufweisen. Für die automatisierte optische Erfassung des Zielgebiets kann die endoskopische Stereooptik außerdem eine Kamera aufweisen oder mit einer solchen Kamera gekoppelt sein. Die Kamera kann beispielsweise eine Hochgeschwindigkeits-Digitalkamera oder eine Kamera eines Mobiltelefons sein.

Die endoskopische Stereooptik kann ferner eine integrierte Beleuchtungseinrichtung aufweisen, z. B. LED- oder LD-Lichtquellen. Dies ermöglicht eine optimale Beleuchtung des Zielgebiets, wobei die Beleuchtung mit unterschiedlichen Lichtstärken erfolgen kann, je nach Betriebszustand der medizinischen Vorrichtung. So kann beispielsweise bei Anzeige eines Live-Bilds der Kamera weniger Licht oder gar kein Licht von der Beleuchtungseinrichtung abgestrahlt werden, da in diesem Betriebszustand in der Regel eine größere Belichtungszeit der Kamera zur Verfügung steht. Bei einer Bildaufnahme mit einer sehr hohen Aufnahmefrequenz steht dagegen eine geringere Belichtungszeit zur Verfügung. In diesem Betriebszustand kann daher die Beleuchtungseinrichtung zur Abgabe einer höheren Lichtstärke angesteuert werden. Gemäß einer vorteilhaften Weiterbildung der Erfindung ist vorgesehen, dass die endoskopische Stereooptik an einem distalen Ende Lichtstrahl-Ablenkungsoptiken zur Richtungsablenkung aufgenommener Lichtstrahlen in einem vorgegebenen Winkel aufweist, insbesondere in einem im Wesentlichen rechten Winkel. Die Lichtstrahl-Ablenkungsoptiken können beispielsweise als Prisma oder als Spiegel ausgebildet sein. Durch die Ablenkungsoptiken kann auch ein Zielgebiet am Lebewesen optisch erfasst werden, das sich in einer Körperöffnung oder Körperhöhle befindet, wie z. B. im Rachenraum.

Gemäß einer vorteilhaften Weiterbildung der Erfindung ist vorgesehen, dass die medizinische Vorrichtung eine automatische Stimulationsantworterfassungseinrichtung aufweist, die zur automatischen Erkennung einer durch einen im Zielgebiet aufgetroffenen Fluidtropfens ausgelösten Stimulationsantwort, z.B. ein Reflex, des Lebewesens eingerichtet ist. Auf diese Weise kann ohne manuelle Tätigkeiten die ausgelöste Stimulationsantwort des Lebewesens identifiziert werden. Damit kann automatisch die Latenzzeit bestimmt werden.

Gemäß einer vorteilhaften Weiterbildung der Erfindung ist vorgesehen, dass die medizinische Vorrichtung eine automatische Latenzzeiterfassungseinrichtung aufweist, die zur automatischen Bestimmung der Latenzzeit einer durch einen im Zielgebiet aufgetroffenen Fluidtropfens ausgelösten Stimulationsantwort des Lebewesens eingerichtet ist. Auch hierdurch wird der Anwender von manuellen Tätigkeiten entlastet. Die Latenzzeiterfassungseinrichtung kann beispielsweise anhand optisch erfasster Daten den Stimulationszeitpunkt automatisch bestimmen, indem der Auftreffzeitpunkt des Fluidtropfens im Zielgebiet bestimmt wird.

Gemäß einer vorteilhaften Weiterbildung der Erfindung ist vorgesehen, dass die Tropfenabschuss-Vorrichtung wenigstens einen Drucksensor zur Sensierung des Fluiddrucks des Fluides, aus dem der abzuschießende Fluidtropfen erzeugt wird, aufweist. Durch den Drucksensor kann sichergestellt werden, dass das Fluid mit einem vorbestimmten Druck bereit gestellt wird und hierdurch der Fluidtropfen mit einer definierten Mündungsenergie abgeschossen wird. Ein von dem Drucksensor erzeugtes Drucksignal kann zudem der Flugbahn-Prädiktionseinrichtung als Eingangssignal zugeführt werden.

Gemäß einer vorteilhaften Weiterbildung der Erfindung ist vorgesehen, dass die Flugbahn-Prädiktionseinrichtung zur Prädiktion der Flugbahn eines Fluidtropfens anhand von gespeicherten Flugbahn-Charakterisierungsdaten, die die Flugbahn eines Fluidtropfens bei bekannten Systemparametern, wie z.B. Düsengeometrie, Systemdruck und Ventilöffnungszeit, charakterisieren, eingerichtet ist. Dies erlaubt eine einfache und zuverlässige automatische Prädiktion der Flugbahn des Fluidtropfens. Die gespeicherten Flugbahn-Charakterisierungsdaten können beispielsweise in einem vorhergehenden Vorbereitungsschritt erfasst werden, in dem die medizinische Vorrichtung noch nicht aktiv an einem Lebewesen eingesetzt wird. Beispielsweise können die Flugbahn-Charakterisierungsdaten unter idealen Laborbedingungen bestimmt werden, z.B. in einer der Anwendung entsprechenden Lage des Systems (Endoskopschaft horizontal, Tropfenschuss mit der Schwerkraft).

Gemäß einer vorteilhaften Weiterbildung der Erfindung ist vorgesehen, dass das Fluid, aus dem der abzuschießende Fluidtropfen erzeugt wird, wasserbasiert ist und wenigstens einen die Oberflächenspannung des Fluidtropfens erhöhenden Zusatzstoff aufweist. Auf dieses Weise kann der Fluidtropfen an einem Lebewesen ohne Gesundheitsgefährdung eingesetzt werden. Durch die Erhöhung der Oberflächenspannung mittels des Zusatzstoffs, z. B. durch Salz, insbesondere Kochsalz, kann der Fluidtropfen stabilisiert werden und damit z. B. für die Auslösung einer Stimulationsantwort optimiert werden. Das Fluid kann als Zusatzstoff zudem ein Färbemittel aufweisen, durch das dem Fluid eine gewünschte Färbung gegeben wird. Das Färbemittel kann z.B. eine Lebensmittelfarbe sein. Durch die Färbung des Fluids wird die automatische optische Erfassung des Fluids nach Auftreffen des Fluidtropfens im Zielgebiet verbessert.

Gemäß einer vorteilhaften Weiterbildung der Erfindung weist die medizinische Vorrichtung eine Atmungserfassungsvorrichtung auf, die zur Erfassung der Atemphasen des Lebewesens eingerichtet ist, wobei ein die Atemphasen charakterisierendes Signal der Atmungserfassungsvorrichtung der Tropfenabschuss-Vorrichtung als Triggersignal zur Triggerung des Abschließens eines Fluidtropfens zugeführt ist. Auf diese Weise kann ein ungestörter Tropfenflug im Rachenraum eines Lebewesens sichergestellt werden. Es kann insbesondere vermieden werden, dass der abzuschießende Fluidtropfen während einer stärkeren Luftbewegung beispielsweise beim Einatmen oder Ausatmen des Lebewesens erfolgt. Vorteilhafterweise gibt die Atmungserfassungsvorrichtung ein Triggersignal an die Tropfenabschussvorrichtung ab, wenn ein Zeitpunkt mit geringer Luft-Strömungsgeschwindigkeit im Rachen vorliegt, z. B. im Übergang von einer Einatmungsphase zu einer Ausatmungsphase oder einer Ausatmungsphase zu einer Einatmungsphase. Die Atmungserfassungsvorrichtung kann beispielsweise die durch die Atmung hervorgerufenen Brustbewegungen eines Lebewesens erfassen, beispielsweise durch in einen Brustgurt integrierte Messvorrichtungen.

Die eingangs genannte Aufgabe wird außerdem gelöst durch ein Verfahren zur Prädiktion eines Auftrefforts eines mit einer medizinischen Vorrichtung der zuvor erläuterten Art abgeschossenen Fluidtropfens, wobei anhand gespeicherter Flugbahn-Charakterisierungsdaten eine Flugbahn-Trajektorie eines abzuschießenden Fluidtropfens bestimmt wird und unter Nutzung von Bildinformationen des optischen Aufnahmesystems, ggf. dreidimensionalen Bildinformationen, der voraussichtliche Auftreffort des Fluidtropfens im Zielgebiet abgeschätzt wird und überlagert mit einer Darstellung des Zielgebiets auf einem Anzeigegerät angezeigt wird. Auch hierdurch können die zuvor erläuterten Vorteile realisiert werden. Der geschätzte Auftreffort kann beispielsweise durch rechnerische Bestimmung eines Schnittpunkts der aus den Flugbahn-Charakterisierungsdaten bestimmten Flugbahn mit einer Tiefenkarte des Zielgebiets, die durch die endoskopische Stereooptik ermittelt wird, bestimmt werden. Wie erwähnt, können auch andere optische Aufnahmesysteme als die endoskopische Stereooptik zum Einsatz kommen, wie z.B. Time-of-Flight-Verfahren. Bei Anwendung einer für eine dreidimensionale Darstellung geeigneten Anzeigeeinrichtung ist die Bestimmung des Schnittpunkts mittels der Tiefenkarte nicht erforderlich, da die visuelle Darstellung bereits dreidimensional erfolgt.

Gemäß einer vorteilhaften Weiterbildung der Erfindung, die auch als eigenständige Erfindung anzusehen ist, ist vorgesehen, dass nach Abschuss des Fluidtropfens der Auftreffzeitpunkt des Fluidtropfens im Zielgebiet und hierdurch der Stimulationszeitpunkt automatisch bestimmt werden. Der Auftreffzeitpunkt des Fluidtropfens im Zielgebiet kann beispielsweise anhand einer Auswertung von mit der Kamera aufgenommenen Bildern des Zielgebiets erfolgen. Beispielsweise kann der Auftreffzeitpunkt anhand einer optischen Auswertung des globalen optischen Flusses einer Sequenz von Differenzbildern bestimmt werden. Die Bilder können mittels der endoskopischen Stereooptik aufgenommen werden.

Gemäß einer vorteilhaften Weiterbildung der Erfindung ist vorgesehen, dass eine durch einen im Zielgebiet aufgetroffenen Fluidtropfen ausgelöste Stimulationsantwort des Lebewesens automatisch detektiert wird und aus dem Zeitpunkt der Stimulationsantwort und dem Stimulationszeitpunkt die Latenzzeit der Stimulationsantwort bestimmt wird. Auch hierdurch wird der Anwender von manuellen Tätigkeiten bei der Bestimmung der Latenzzeit entbunden.

Die eingangs genannte Aufgabe wird außerdem gelöst durch ein Computerprogramm mit Programmcodemitteln, eingerichtet zur Durchführung eines Verfahrens der zuvor erläuterten Art, wenn das Computerprogramm auf einem Rechner ausgeführt wird. Der Rechner kann beispielsweise ein Mikrocomputer oder Mikrocontroller der medizinischen Vorrichtung sein. Der Rechner kann z. B. Teil der Flugbahn-Prädiktionseinrichtung, der automatischen Stimulationsantworterfassungseinrichtung und/oder der automatischen Latenzzeiterfassungseinrichtung sein.

Die automatische Prädiktion der Flugbahn eines mittels der Tropfenabschuss-Vorrichtung abzuschießenden Fluidtropfens kann beispielsweise wie folgt durchgeführt werden:
1. Nutzung der kalibrierten, endoskopischen Stereooptik mit der Tropfenabschuss-Vorrichtung, die eine starre Verbindung mit der Stereooptik aufweist, zur Erfassung der Tropfenflugbahn im Raum. Dies geschieht außerhalb des Lebewesens in einer ähnlichen Lage der Vorrichtung wie bei der tatsächlichen Untersuchung (Endoskop-Schaft horizontal) durch Aufnahme einer Videosequenz des Tropfenflugs bei hoher Aufnahmefrequenz. Der Tropfenort im Raum wird in jedem Frame für verschiedene Kombinationen von Fluidsystemdruck und Ventilöffnungszeit erfasst. Auf diese Weise wird eine Schar an Tropfenflugbahnen gespeichert. Die Speicherung erfolgt jeweils als Gerade im Raum, die die aufgenommenen Orte des Tropfens auf seiner Trajektorie möglichst gut approximiert.
2. Während der klinischen Stimulationsantwortuntersuchung wird die kalibrierte, endoskopische Stereooptik zur Erstellung einer metrischen Echtzeit-Tiefenkarte des Kehlkopfes genutzt. Die dazu notwendige Erkennung korrespondierender Punkte im linken und rechten Endoskopbild kann beispielsweise durch Anwendung des etablierten "Block Matching"-Algorithmus erreicht werden. Die Tiefenkarte wird aus dem Live-Bild in Form einer Punktwolke berechnet. Gleichzeitig wird dem Untersucher das Live-Bild einer der Optiken der endoskopischen Stereooptik angezeigt, um ihm eine Orientierung im Untersuchungsraum zu ermöglichen.
3. Die vom Untersucher an der medizinischen Vorrichtung wählbaren Werte für Systemdruck und Ventilöffnungszeit entsprechen einer in Schritt 1 gespeicherten Tropfenflugbahn. Diese wird nun aufgerufen. Der Abstand zwischen den Punkten der metrischen Tiefenkarte aus Schritt 2 und der im Vorfeld aufgezeichneten Tropfenflugbahn wird für die aktuelle Lage des Endoskop-Systems berechnet.
4. Es existiert mindestens ein Punkt der metrischen Tiefenkarte aus Schritt 2, an dem sich diese mit der Tropfenflugbahn schneidet bzw. der Abstand minimal ist. Dieser Punkt wird dem Untersucher als voraussichtlicher Auftreffort und damit Stimulationsort im Live-Bild angezeigt.

Beispielsweise kann eine automatisierte Bestimmung der Latenz des laryngealen Adduktionsreflexes anhand einer endoskopisch aufgezeichneten Videosequenz wie folgt durchgeführt werden:
1. Bildvorverarbeitung, z.B. Umwandlung der Bilddaten in Graustufen-Frames, Histogrammausgleich.
2. Erstellung einer Sequenz aus Differenzbildern durch Subtraktion des Durchschnittsbildes der ersten Frames der Sequenz vom aktuellen Frame.
3. Berechnung des globalen optischen Flusses für alle Frames der Differenzbild-Folge mit etablierten Verfahren (z. B. nach Horn-Schunck).
4. Berechnung der Summe der Beträge der Komponentensummen der für jedes Frame berechneten Flussvektoren (definiert als Größe) und Glättung. Normierung durch Division durch größten berechneten Wert der jeweiligen Sequenz.
5. Automatische Detektion des Stimulationszeitpunktes durch Peak Detection, da der Aufprall des Fluidtropfens einen Peak im in Schritt 4 berechneten Signal verursacht. Dies ist möglich, weil das Zerplatzen des Fluidtropfens beim Aufprall einen deutlich sichtbaren, lokalen Anstieg des optischen Flusses verursacht.
6. Nutzung eines mithilfe einer großen Zahl von bereits ausgewerteten Videosequenzen antrainierten neuronalen Netzes zur Ableitung der Reflexlatenz aus dem Verlauf der Größe. Die ermittelte Latenzzeit kann nun als quantitatives Maß für die Reflexgüte genutzt werden. Eine gegenüber dem Durchschnittswert bei gesunden Probanden höhere Reflexlatenz weist auf einen pathologischen Ablauf hin. Die Indikation einer Folgeuntersuchung bzw. die Einteilung in eine Pneumonie-Risikogruppe kann wiederum unter Nutzung der künstlichen Intelligenz (z. B. des maschinellen Lernens) erfolgen.

Die Erfindung wird nachfolgend anhand von Ausführungsbeispielen unter Verwendung von Zeichnungen näher erläutert.

Es zeigen

| | |
|---|---|
| Fig. 1 | - einen Gesamtüberblick über eine erfindungsgemäße medizinische Vorrichtung und |
| Fig. 2 | - eine endoskopische Stereooptik der medizinischen Vorrichtung in einer perspektivischen Darstellung und |
| Fig. 3 | die endoskopische Stereooptik gemäß Figur 2 in einer Explosionsdarstellung und |
| Fig. 4 und 5 | - vergrößerte Detaildarstellungen des distalen Endes einer endoskopischen Stereooptik und |
| Fig. 6 | - eine Atmungserfassungsvorrichtung der medizinischen Vorrichtung und |
| Fig. 7 | - eine Visualisierung des Zielgebiets mittels einer 3D-Brille. |

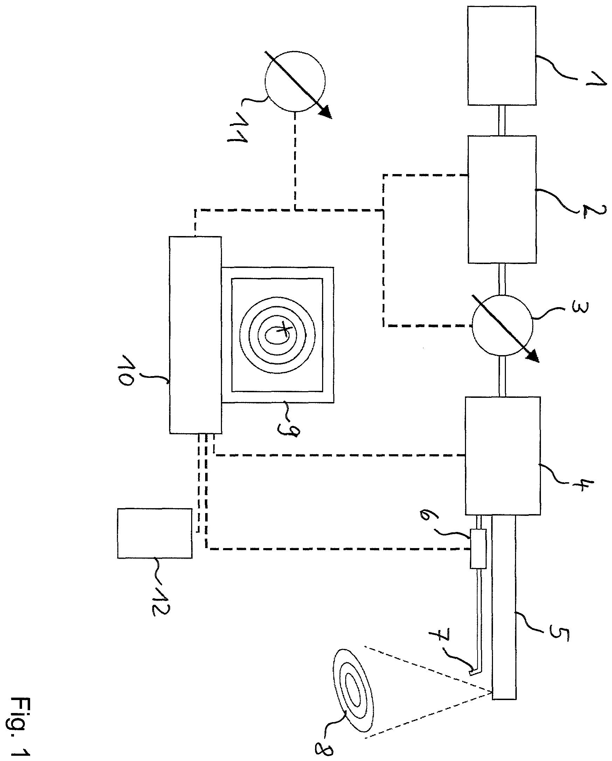

In der Figur 1 sind Fluidleitungen in durchgezogenen Linien dargestellt, elektrische Signalleitungen mit gestrichelten Linien. Erkennbar ist ein Fluidreservoir 1, in dem eine Menge des Fluids, aus dem ein abzuschießender Fluidtropfen erzeugt werden soll, gespeichert ist. An das Fluidreservoir 1 ist eine Pumpe 2 angeschlossen, beispielsweise eine Spritzenpumpe. Die Pumpe 2 kann das Fluid aus dem Fluidreservoir 2 mit einem bestimmten Druck ausgangsseitig bereitstellen. Auf der Ausgangsseite der Pumpe 2 ist ein Drucksensor 3 angeordnet, mit dem der Druck des von der Pumpe 2 abgegebenen Fluids erfasst werden kann.

Das auf diese Weise unter Druck gesetzte Fluid wird einer Handhabungsvorrichtung, die von einem Anwender zu bedienen ist, zugeführt. Die Handhabungsvorrichtung weist eine Kamera 4, eine mit der Kamera 4 verbundene endoskopische Stereooptik 5, ein elektrisch steuerbares Ventil 6 sowie eine Auslassdüse 7 zur Abgabe des Fluidtropfens auf. Die Kamera 4 kann über die Stereooptik 5 ein Zielgebiet 8 am Lebewesen optisch erfassen.

Die medizinische Vorrichtung gemäß Figur 1 weist außerdem eine Atmungserfassungseinrichtung 11, eine Anzeigeeinrichtung 9, beispielsweise in Form eines Bildschirms, eine Steuer- und Datenverarbeitungseinheit 10 sowie ein Pedal 12 auf. Die Steuer- und Datenverarbeitungseinheit 10 beinhaltet die zuvor erwähnte Flugbahn-Prädiktionseinrichtung, die automatische Stimulationsantworterfassungseinrichtung sowie eine Latenzzeiterfassungseinrichtung. Die Steuer- und Datenverarbeitungseinheit 10 erhält als Eingangssignale ein Triggersignal der Atmungserfassungseinrichtung 11, ein Drucksignal vom Drucksensor 3, ein Auslösesignal vom Pedal 12 sowie die über die Kamera 4 erfassten Bilder. Die Steuer- und Datenverarbeitungseinheit 10 steuert die Pumpe 2 sowie das Ventil 6 derart an, dass zu einem gewünschten Zeitpunkt ein Fluidtropfen aus der Düse 7 abgeschossen wird, wobei die Pumpe 2 derart angesteuert wird, dass der erzeugte Fluiddruck, der am Drucksensor 3 gemessen wird, einen gewünschten Sollwert erreicht. Zudem wird eine definierte Ventilöffnungszeit automatisch eingestellt. Die Steuer- und Datenverarbeitungseinheit 10 wertet zudem die Bilder der Kamera 4 aus und erzeugt die Tiefenkarte des Zielgebiets 8. Aus den erfassten Daten wird über die Flugbahn- Prädiktionseinrichtung die Flugbahn eines abzuschießenden Fluidtropfens bestimmt und auf der Anzeigeeinrichtung 9 durch einen prädizierten Auftreffort des Fluidtropfens im Zielgebiet visualisiert. Hierbei wird der Auftreffort überlagert mit einer Darstellung des Zielgebiets 8 auf dem Anzeigegerät 9 wiedergegeben werden.

Wird vom Anwender mittels des Pedals 12 das Kommando zum Abschießen eines Fluidtropfens gegeben, so steuert die Steuer- und Datenverarbeitungseinheit 10 das Ventil 6 auf Durchlass, wenn ein Triggersignal von der Atmungserfassungseinrichtung 11 empfangen wird. Das Ventil 6 wird nur kurzzeitig auf Durchlass geschaltet und danach wieder geschlossen, so dass ein relativ kleiner Fluidtropfen aus der Düse 7 in das Zielgebiet 8 geschossen wird. Der Fluidtropfen ist über Ventilöffnungszeit und Systemdruck in seiner Größe und Geschwindigkeit variierbar.

Das Auslösesignal zum Abschuss des Fluidtropfens kann auch auf andere Weise als durch das erwähnte Pedal 12 erfolgen, z.B. durch eine manuelle Betätigungseinrichtung oder durch akustische Signalerfassung, z.B. durch ein Spracherkennungsmodul.

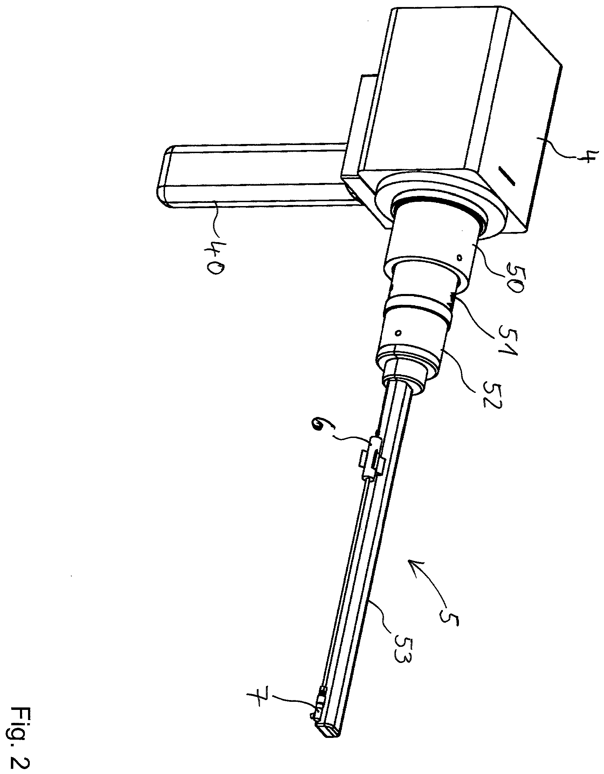

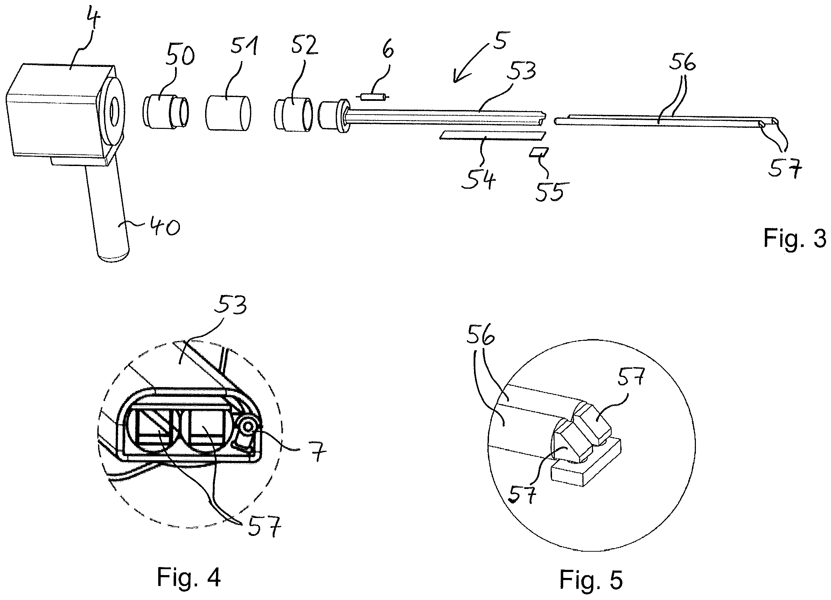

Die Figur 2 und 3 zeigen die Handhabungsvorrichtung mit den anhand der Figur 1 erläuterten Komponenten. Erkennbar ist die Kamera 4, die beispielsweise einen Handgriff 40 aufweist. An der Kamera 4 kann ein proximaler Adapter 50, daran eine Vergrößerungsoptik 51 und daran ein distaler Adapter 52 montiert werden. Am distalen Adapter 52 kann eine Optikhalterung 53 montiert werden. In oder an der Optikhalterung 53 kann das Ventil 6 angeordnet sein. Entlang der Optikhalterung 53 verläuft ein Fluidkanal, der am distalen Ende der Optikhalterung 53 an der Düse 7 endet.

Die Optikhalterung 53 weist Aufnahmekanäle für zwei Endoskop-Optiken 56 auf, z. B. Lichtleiter oder Stablinsenoptiken, durch die die endoskopische Stereooptik gebildet wird. An dem distalen Ende der Endoskop-Optiken 56 können Lichtstrahl-Ablenkungsoptiken 57 angeordnet sein, durch die die optische Erfassungsrichtung beispielsweise etwa rechtwinklig zur Längserstreckung der Endoskop-Optiken 56 umgelenkt wird.

An oder in der Optikhalterung 53 kann eine Beleuchtungseinrichtung angeordnet sein, beispielsweise mit Lichtquellen 55 in Form von LEDs oder Laserdioden. Zusätzlich kann ein Kühlkörper 54 zur Kühlung der Lichtquellen 55 dort angeordnet sein. Die Beleuchtung des Untersuchungsgebietes kann auch mit Lichtleitfasern erfolgen, in die außerhalb des Endoskopschafts eine externe Lichtquelle eingekoppelt ist.

Das Ventil 6 kann bspw. über einen Halteclip seitlich an der Optikhalterung 53 befestigt sein.

Wie die Figur 4 zeigt, können die Endoskop-Optiken 56 innerhalb der Optikhalterung 53 untergebracht sein und am Ende die Lichtstrahl-Ablenkungsoptiken 57 aufweisen. Die Figur 5 zeigt diesen distalen Bereich der Endoskop-Optiken 56 ohne die umhüllende Optikhalterung 53.

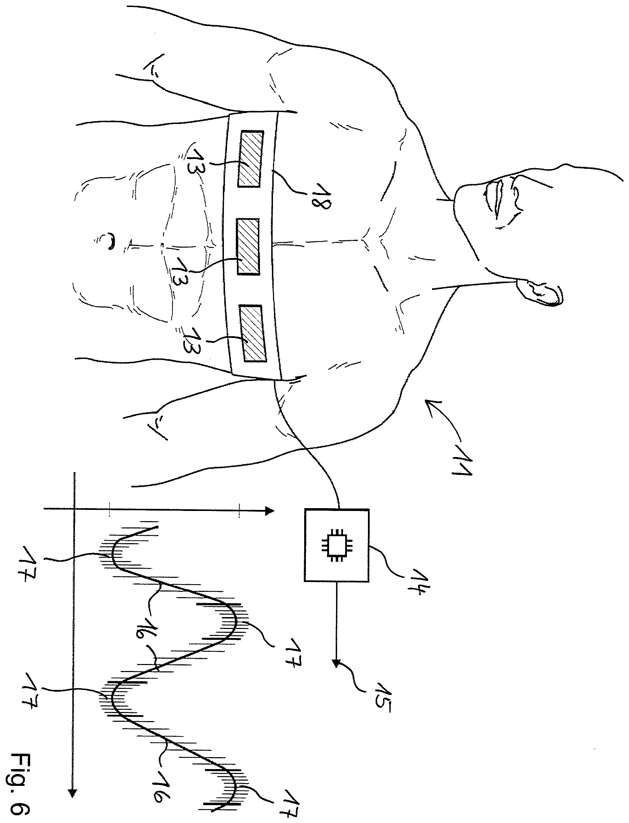

Die Figur 6 zeigt die Atmungserfassungseinrichtung 11 bei Anwendung an einem Lebewesen. Die Atmungserfassungseinrichtung 11 weist in dieser Ausführungsform einen Brustgurt 12 auf, der um die Brust des Lebewesens gelegt ist. An dem Brustgurt 12 sind Dehnungsmessvorrichtungen 13 angeordnet, die beispielsweise in den Brustgurt 12 integriert sein können. Über die Dehnungsmessvorrichtungen 13 wird der aktuelle Dehnungszustand der Brust des Lebewesens erfasst und als elektrisches Signal an eine Auswerteelektronik 14 abgegeben. Die Auswerteelektronik 14, die beispielsweise einen Mikrokontroller aufweisen kann, wertet die Signale der Dehnungsmessvorrichtungen 13 aus und bestimmt hieraus Bereiche, in denen nur eine geringe Luftströmung aufgrund der Atmung des Lebewesens zu erwarten ist. In solchen Phasen geringer Luftströmung gibt die Auswerteelektronik 14 das Triggersignal 15 an die Steuer- und Datenverarbeitungseinheit 10 ab.

Der in der Figur 6 rechts unten dargestellte beispielhafte zeitliche Signalverlauf des durch die Dehnungsmessvorrichtungen 13 gemessenen Signals gibt die Einatmungs- und Ausatmungsphasen des Lebewesens wieder. In Phasen mit starkem Signalanstieg oder starkem Signalabfall, d. h. den Phasen 16, würde ein abgeschossener Fluidtropfen durch die Luftströmung der Atmung zu sehr gestört werden. Daher erkennt die Auswerteelektronik 14 die Phasen 17 mit geringer Luftströmung und gibt in diesen Phasen 17 das Triggersignal 15 ab.

Die verwendeten Messelemente können statt der Dehnungsmessstreifen 13 auch andere Messelemente sein, z. B. piezoelektrische Elemente, rotatorische Encoder mit Seilzug oder ähnliches. Der gewonnene Messwert ist ein Maß für die Volumenänderung des Brustkorbs. Die Phasen 17 können beispielsweise als Zeitbereiche um den Minimalwert und den Maximalwert im zeitlichen Verlauf des gemessenen Signals detektiert werden.

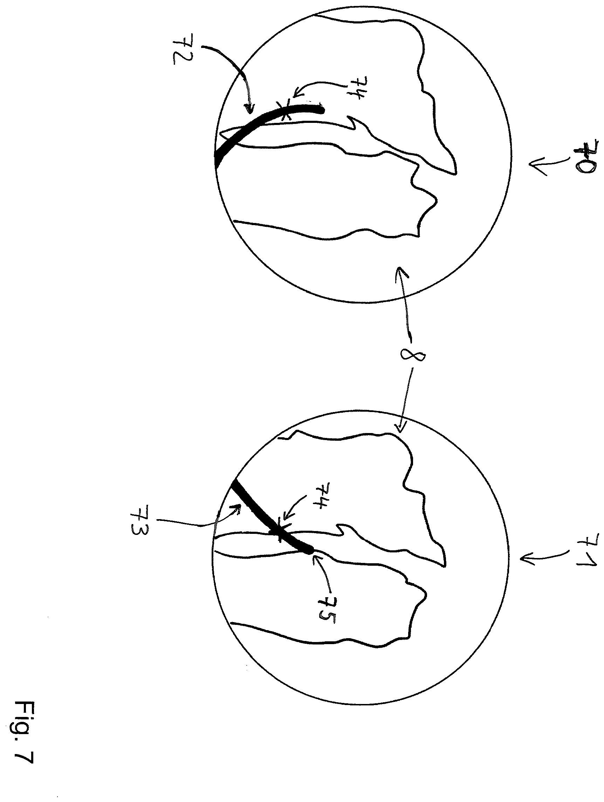

Anhand der Figur 7 soll eine Ausführungsform der Erfindung erläutert werden, bei der nicht die erläuterte Tiefenkarte mittels des optischen Aufnahmesystems ermittelt wird, sondern eine zur dreidimensionalen Darstellung geeignete Anzeigeeinrichtung verwendet wird, was in diesem Fall am Beispiel einer 3D-Brille erläutert werden soll. Es erfolgt eine stereoskopische Beobachtung des Zielgebiets 8 durch einen linken Visualisierungsbereich 70 und einen rechten Visualisierungsbereich 71 der 3D-Brille. Durch Anzeigeelemente der 3D-Brille wird die Flugbahn des Fluidtropfens im linken Visualisierungsbereich 70 als Flugbahn 72 und im rechten Visualisierungsbereich 71 als Flugbahn 73 eingeblendet. Die realen Flugbahnen der visualisierten Flugbahnen 72, 73 enden am wahrgenommenen Schnittpunkt mit dem Zielgebiet, der dem prädizierten Auftreffort 74 des Fluidtropfens entspricht und vor dem Ende 75 der visualisierten Flugbahnen 72, 73 liegen kann. Die Prädiktion der beiden dargestellten Flugbahnen 72, 73 erfolgt dabei für einen definierten Parametersatz, der z.B. durch die Mündungsenergie der Abschussvorrichtung, die bspw. durch den Druck und die Ventilöffnungszeit definiert ist, sowie die Gravitationsrichtung festgelegt ist. In diese Ausführungsform kann auch ohne die Tiefenkarte dem Benutzer der Auftreffort 74 visualisiert werden, was durch die Betrachtung des linken und des rechten Visualisierungsbereiches 70, 71 erfolgt und damit den "richtigen" Eindruck des Auftrefforts 74 ergibt.

## Patentansprüche

1. Medizinische Vorrichtung zum gezielten Abschuss eines Fluidtropfens in ein Zielgebiet (8) eines Lebewesens, wobei die medizinische Vorrichtung aufweist:
a) ein optisches Aufnahmesystem (5) zur automatisierten optischen Erfassung des Zielgebiets (8),
b) eine mechatronisch regelbare oder geregelte Tropfenabschuss-Vorrichtung (2, 3, 6, 7), durch die der Fluidtropfen abschießbar ist,
c) eine Flugbahn-Prädiktionseinrichtung, die zur Prädiktion der Flugbahn eines mittels der Tropfenabschuss-Vorrichtung (2, 3, 6, 7) abzuschießenden Fluidtropfens sowie zur Anzeige des voraussichtlichen Auftreffortes des Fluidtropfens im Zielgebiet eingerichtet ist,
**dadurch gekennzeichnet, dass** die Flugbahn-Prädiktionseinrichtung einen Rechner aufweist und dazu eingerichtet ist, rechnergesteuert anhand gespeicherter Flugbahn-Charakterisierungsdaten eine Flugbahn-Trajektorie des abzuschießenden Fluidtropfens zu bestimmen und unter Nutzung von Bildinformationen des optischen Aufnahmesystems (5) einen Auftreffbereich des Fluidtropfens im Zielgebiet (8) abzuschätzen und überlagert mit einer Darstellung des Zielgebiets auf einem Anzeigegerät (9) anzuzeigen.

2. Medizinische Vorrichtung nach Anspruch 1, **dadurch gekennzeichnet, dass** das optische Aufnahmesystem (5) an einem distalen Ende Lichtstrahl-Ablenkungsoptiken (57) zur Richtungsablenkung aufgenommener Lichtstrahlen in einem vorgegebenen Winkel aufweist, insbesondere in einem im Wesentlichen rechten Winkel.

3. Medizinische Vorrichtung nach einem der vorhergehenden Ansprüche, **dadurch gekennzeichnet, dass** die medizinische Vorrichtung eine automatische Stimulationsantworterfassungseinrichtung aufweist, die zur automatischen Erkennung einer durch einen im Zielgebiet (8) aufgetroffenen Fluidtropfens ausgelösten Stimulationsantwort des Lebewesens eingerichtet ist.

4. Medizinische Vorrichtung nach Anspruch 3, **dadurch gekennzeichnet, dass** die medizinische Vorrichtung eine automatische Latenzzeiterfassungseinrichtung aufweist, die zur automatischen Bestimmung der Latenzzeit eines durch einen im Zielgebiet (8) aufgetroffenen Fluidtropfens ausgelösten Stimulationsantwort des Lebewesens eingerichtet ist.

5. Medizinische Vorrichtung nach einem der vorhergehenden Ansprüche, **dadurch gekennzeichnet, dass** die Tropfenabschuss-Vorrichtung (2, 3, 6, 7) wenigstens einen Drucksensor (3) zur Sensierung des Fluiddrucks des Fluides, aus dem der abzuschießende Fluidtropfen erzeugt wird, aufweist.

6. Medizinische Vorrichtung nach einem der vorhergehenden Ansprüche, **dadurch gekennzeichnet, dass** die Flugbahn-Prädiktionseinrichtung zur Prädiktion der Flugbahn eines Fluidtropfens anhand von gespeicherten Flugbahn-Charakterisierungsdaten, die die Flugbahn eines Fluidtropfens bei bekannten Systemparametern charakterisieren, eingerichtet ist.

7. Medizinische Vorrichtung nach einem der vorhergehenden Ansprüche, **dadurch gekennzeichnet, dass** das Fluid, aus dem der abzuschießende Fluidtropfen erzeugt wird, wasserbasiert ist und wenigstens einen die Oberflächenspannung des Fluidtropfens erhöhenden Zusatzstoff aufweist.

8. Medizinische Vorrichtung nach einem der vorhergehenden Ansprüche, **dadurch gekennzeichnet, dass** die medizinische Vorrichtung eine Atmungserfassungsvorrichtung (11) aufweist, die zur Erfassung der Atemphasen (16, 17) des Lebewesens eingerichtet ist, wobei ein die Atemphasen (16, 17) charakterisierendes Signal der Atmungserfassungsvorrichtung (11) der Tropfenabschuss-Vorrichtung (2, 3, 6, 7) als Triggersignal (15) zur Triggerung des Abschließens eines Fluidtropfens zugeführt ist.

9. Verfahren zur Prädiktion eines Auftrefforts eines mit einer Vorrichtung nach einem der vorhergehenden Ansprüche abgeschossenen Fluidtropfens, **dadurch gekennzeichnet, dass** anhand gespeicherter Flugbahn-Charakterisierungsdaten eine Flugbahn-Trajektorie eines abzuschießenden Fluidtropfens bestimmt wird und unter Nutzung von Bildinformationen des optischen Aufnahmesystems (5) ein Auftreffbereich des Fluidtropfens im Zielgebiet (8) abgeschätzt wird und überlagert mit einer Darstellung des Zielgebiets auf einem Anzeigegerät (9) angezeigt wird.

10. Verfahren zur Bestimmung eines Auftreffzeitpunkts eines mit einer Vorrichtung nach einem der vorhergehenden Ansprüche abgeschossenen Fluidtropfens, insbesondere Verfahren nach Anspruch 9, **dadurch gekennzeichnet, dass** nach Abschuss des Fluidtropfens der Auftreffzeitpunkt des Fluidtropfens im Zielgebiet (8) und hierdurch der Stimulationszeitpunkt automatisch bestimmt werden.

11. Verfahren nach Anspruch 10, **dadurch gekennzeichnet, dass** eine durch einen im Zielgebiet (8) aufgetroffenen Fluidtropfen ausgelöste Stimulationsantwort des Lebewesens automatisch detektiert wird und aus dem Zeitpunkt der Stimulationsantwort und dem Stimulationszeitpunkt die Latenzzeit der Stimulationsantwort bestimmt wird.

12. Computerprogramm mit Programmcodemitteln, eingerichtet zur Durchführung eines Verfahrens nach einem der Ansprüche 9 bis 11, wenn das Computerprogramm auf einem Rechner ausgeführt wird.

## Claims

1. A medical device for selectively firing a drop of fluid into a target area (8) of a living being, the medical device comprising
a) an optical imaging system (5) for automated optical detection of the target area (8),
b) a mechatronically controllable or regulated droplet launching device (2, 3, 6, 7) by which the fluid droplet can be launched,
c) a trajectory prediction device which is set up to predict the trajectory of a fluid drop to be launched by means of the drop launching device (2, 3, 6, 7) and to indicate the expected impact of the fluid drop in the target area, **characterized in that** the trajectory prediction device has a computer and is set up to determine, under computer control, a trajectory of the fluid drop to be fired using stored trajectory characterization data and to estimate an impact area of the fluid drop in the target area (8) using image information from the optical imaging system (5) and to display it superimposed on a display device (9) with a representation of the target area.

2. Medical device according to claim 1, **characterized in that** the optical imaging system (5) has light beam deflection optics (57) at a distal end for directional deflection of recorded light beams at a predetermined angle, in particular at a substantially right angle.

3. Medical device according to one of the preceding claims, **characterized in that** the medical device has an automatic stimulation response detection device which is set up for automatic recognition of a stimulation response of the living being triggered by a fluid drop which has hit the target area (8).

4. Medical device according to claim 3, **characterized in that** the medical device has an automatic latency time detection device which is set up to automatically determine the latency time of a stimulation response of the living being triggered by a fluid drop which has hit the target area (8).

5. Medical device according to one of the preceding claims, **characterized in that** the drop-launching device (2, 3, 6, 7) comprises at least one pressure sensor (3) for sensing the fluid pressure of the fluid from which the fluid drop to be launched is produced.

6. Medical device according to any of the preceding claims, **characterized in that** the trajectory prediction device is adapted to predict the trajectory of a fluid drop from stored trajectory characterization data characterizing the trajectory of a fluid drop at known system parameters.

7. Medical device according to any of the foregoing claims, **characterized in that** the fluid from which the fluid drop to be shot is produced is water-based and has at least one additive increasing the surface tension of the fluid drop.

8. A medical device according to one of the preceding claims, **characterized in that** the medical device comprises a respiration detection device (11) which is adapted to detect the respiration phases (16, 17) of the living being, wherein a signal characterizing the respiration phases (16, 17) of the respiration detection device (11) is supplied to the drop-lauding device (2, 3, 6, 7) as a trigger signal (15) for triggering the launching of a fluid drop.

9. Method for predicting an impact location of a fluid drop fired with a device according to one of the preceding claims, **characterized in that** a trajectory of a fluid drop to be fired is determined with the aid of stored trajectory characterization data and, using image information of the optical imaging system (5), an impact area of the fluid drop in the target area (8) is estimated and displayed superimposed with a representation of the target area on a display device (9).

10. Method for determining a moment of impact of a fluid drop launched with a device according to one of the preceding claims, in particular method according to claim 9, **characterized in that** after the fluid drop has been launched, the moment of impact of the fluid drop in the target area (8) and thereby the stimulation moment are automatically determined.

11. Method according to claim 10, **characterized in that** a stimulation response of the living being triggered by a fluid drop hitting the target area (8) is automatically detected and the latency time of the stimulation response is determined from the time of the stimulation response and the stimulation time.

12. A computer program with program code means adapted to perform a process according to one of claims 9 to 11 when the computer program is executed on a computer

## Revendications

1. Dispositif médical pour le tir ciblé d'une gouttelette de fluide dans une zone cible (8) d'un être vivant, le dispositif médical comprenant :
a) un système d'enregistrement optique (5) pour l'acquisition optique automatisée de la zone cible (8),
b) un dispositif de tir de gouttelettes (2, 3, 6, 7) régulé ou susceptible d'être régulé par voie mécatronique, par lequel la gouttelette de fluide peut être tirée,
c) un moyen de prédiction de trajectoire conçu pour prédire la trajectoire d'une gouttelette de fluide à tirer au moyen du dispositif de tir de gouttelettes (2, 3, 6, 7) et pour afficher le lieu prévu de l'impact de la gouttelette de fluide dans la zone cible,
**caractérisé en ce que**
le moyen de prédiction de trajectoire comprend un calculateur et est conçu pour déterminer, sous contrôle informatique, une trajectoire de la gouttelette de fluide à tirer, en utilisant des données de caractérisation de trajectoire stockées, et pour estimer une zone d'impact de la gouttelette de fluide dans la zone cible (8), en utilisant des informations d'image du système d'enregistrement optique (5), et pour l'afficher en superposition avec une représentation de la zone cible sur un appareil d'affichage (9).

2. Dispositif médical selon la revendication 1,
**caractérisé en ce que**
le système d'enregistrement optique (5) comprend des optiques de déviation de faisceau lumineux (57) à une extrémité distale pour la déviation directionnelle des faisceaux lumineux enregistrés à un angle prédéterminé, en particulier à un angle sensiblement droit.

3. Dispositif médical selon l'une des revendications précédentes,
**caractérisé en ce que**
le dispositif médical comprend un moyen de détection automatique de la réponse de stimulation conçu pour reconnaître automatiquement une réponse de stimulation de l'être vivant déclenchée par une gouttelette de fluide arrivée dans la zone cible (8).

4. Dispositif médical selon la revendication 3,
**caractérisé en ce que**
le dispositif médical comprend un moyen de détection automatique de temps de latence conçu pour déterminer automatiquement le temps de latence d'une réponse de stimulation dudit être vivant déclenchée par une gouttelette de fluide arrivée dans la zone cible (8).

5. Dispositif médical selon l'une des revendications précédentes,
**caractérisé en ce que**
le dispositif de tir de gouttelettes (2, 3, 6, 7) comprend au moins un capteur de pression (3) pour détecter la pression du fluide à partir duquel la gouttelette de fluide à tirer est générée.

6. Dispositif médical selon l'une des revendications précédentes,
**caractérisé en ce que**
le dispositif de prédiction de trajectoire est conçu pour prédire la trajectoire d'une gouttelette de fluide sur la base de données de caractérisation de trajectoire stockées caractérisant la trajectoire d'une gouttelette de fluide en fonction de paramètres de système connus.

7. Dispositif médical selon l'une des revendications précédentes,
**caractérisé en ce que**
le fluide à partir duquel la gouttelette de fluide à tirer est générée est à base aqueuse et comprend au moins un additif augmentant la tension de surface de la gouttelette de fluide.

8. Dispositif médical selon l'une des revendications précédentes,
**caractérisé en ce que**
le dispositif médical comprend un dispositif de détection de respiration (11) conçu pour détecter les phases respiratoires (16, 17) de l'être vivant, et un signal caractérisant les phases respiratoires (16, 17) provenant du dispositif de détection de respiration (11) est fourni au dispositif de tir de gouttelettes (2, 3, 6, 7) en tant que signal de déclenchement (15) pour déclencher le tir d'une gouttelette de fluide.

9. Procédé pour prédire un lieu d'impact d'une gouttelette de fluide tirée par un dispositif selon l'une des revendications précédentes,
**caractérisé en ce que**
une trajectoire d'une gouttelette de fluide à tirer est déterminée sur la base de données de caractérisation de trajectoire stockées, et une zone d'impact de la gouttelette de fluide dans la zone cible (8) est estimée en utilisant des informations d'image du système d'enregistrement optique (5) et est affichée en superposition à une représentation de la zone cible sur un appareil d'affichage (9).

10. Procédé pour déterminer un instant d'impact d'une gouttelette de fluide tirée par un dispositif selon l'une des revendications précédentes,
en particulier procédé selon la revendication 9,
**caractérisé en ce que**
après le tir de la gouttelette de fluide, l'instant d'impact de la gouttelette de fluide dans la zone cible (8) et, par conséquent, l'instant de stimulation sont déterminés automatiquement.

11. Procédé selon la revendication 10,
**caractérisé en ce que**
une réponse de stimulation de l'être vivant déclenchée par une gouttelette de fluide arrivée dans la zone cible (8) est détectée automatiquement, et le temps de latence de la réponse de stimulation est déterminé à partir de l'instant de la réponse de stimulation et de l'instant de stimulation.

12. Programme informatique comprenant des moyens de code de programme, conçu pour mettre en oeuvre un procédé selon l'une des revendications 9 à 11, lorsque le programme informatique est exécuté sur un ordinateur.
